# EUROPEAN PATENT SPECIFICATION

(11) **EP 4 138 580 B1**
(45) Date of publication and mention of the grant of the patent: **28.02.2024**
(21) Application number: 21725001.8
(22) Date of filing: 13.04.2021
(51) Int. Cl.: A23L 33/105, A61K 31/375, A61K 33/20, A61K 36/185, A61K 36/28, A61K 36/282, A61K 36/288, A61K 36/31, A61K 36/315, A61K 36/484, A61K 36/54, A61K 36/734, A61K 36/74, A61K 36/9068

(54) **ANTI-VIRAL, ANTI-MICROBIAL AND STIMULATING IMMUNITY FOOD SUPPLEMENT**
ANTIVIRALES, ANTIMIKROBIELLES UND STIMULIERENDES IMMUNITÄTSNAHRUNGSMITTELERGÄNZUNGSMITTEL
COMPLÉMENT ALIMENTAIRE ANTIVIRAL, ANTIMICROBIEN ET STIMULANT L'IMMUNITÉ

(30) Priority: 24.04.2020 BG 491420
(43) Date of publication of application: 01.03.2023
(73) Proprietor: Angelov, Patrick Todorov, 1632 Sofia (BG); Stoychev, Kamen Stoychev, 1606 Sofia (BG); McLeay, Nevan John, 1606 Sofia (BG); Spasov, Ivo Petrov, 1715 Sofia (BG)
(72) Inventor: STOYCHEV, Kamen Stoychev, 1606 Sofia (BG); MCLEAY, Nevan John, 1606 Sofia (BG); SPASOV, Ivo Petrov, 1715 Sofia (BG)
(74) Representative: Manev, Kostadin Chanev
(86) International application number: PCT/BG2021/000013
(87) International publication number: WO 2021/212189

(56) References cited:
- EP-A1- 1 602 354
- EP-B1- 1 602 354
- WO-A2-2014/170505
- CN-B- 102 578 397
- KR-A- 20160 112 822

## Description

### Field of technology

This utility model refers to an anti-viral, anti-microbial and stimulating immunity food supplement, which will find application both in preventive health care and to improve the body's immune defences.

### Background of the invention

The human body has many protective barriers that protect us from pathogens. Their entry into it is hampered by various obstacles that our body has created - sneezing, coughing, saliva, skin, stomach acid and others. After the pathogens overcoming these protective barriers, the immune system is included in the defence. The human immune system has to deal with many stressors on a daily basis. It can be weakened by diseases, toxins, air pollution, unhealthy foods and beverages, stress, aging and even physical exertion. In addition, the immune system changes throughout our lives, at every stage of it, depending on age, lifestyle, eating habits and exercise. With overall weakening of the body, pathogens much easier overcome non-specific barriers of protection and penetrate it. People whose immune systems do not work optimally have more infections each year and it takes longer to recover. That is why it is necessary to strengthen the immune system in a timely manner.

Immunostimulants aim to increase the body's defences and have a role in the treatment of pre-existing infections. With their help the period of illness is shortened, recovery after viral and bacterial infections is facilitated.

WO2014/170505 A2 discloses a composition for immunity regulation and/or stimulation, comprising zinc, vitamin C and at least 2 plant extracts.

### Technical essence of the utility model

The task of the utility model is to create an anti-viral, anti-microbial and stimulating immunity food supplement, which will support the functions of the immune system to increase the body's defences.

The problem was solved by creating a food supplement to stimulate immunity, which includes the following components in weight percentages: liquorice - *Glycyrrhiza glabra* from 1.19% to 47.61%, sweet wormwood - *Artemisia annua* from 1.19% to 47, 61%, quinine bark extract-*Cinchona* from 1,19% to 47,61%, hawthorn extract - *Crataegus* from 1,19% to 47,61%, dandelion extract - *Taraxcum* from 1,19% to 47,61%, Vitamin C - Ascorbic acid from 1.19% to 47.61%, DD-Galacturonic acid from 1.19% to 47.61%, apple pectin from 1.19% to 47.61%, bitter orange - *Citrus aurantium* from 1.19% to 47.61%, ginger - *Zingiber officinale* from 0.23% to 11.9%, *Radix isatidis* from 0.23% to 11.9%, *Lindera aggregate* from 0.23 % to 11.9%, woad - *Isatis indigotica* from 0.23% to 9.52%, *Torreya nucifera* from 0.23% to 9.52%, geranium extract - *Pelargonium* from 0.23% to 9.52%, Zinc from 0.23% to 2.38% and Sodium Selenite from 0.00119% to 0.04762%.

In a preferred embodiment of the food supplement, liquorice - *Glycyrrhiza glabra* is 11.91%, sweet wormwood - *Artemisia annua* is 11.91%, quinine bark extract - *Cinchona* is 11.91%, hawthorn extract - *Crataegus* is 9.52%, Dandelion extract - *Taraxcum* is 9.52%, vitamin C - Ascorbic acid is 9.52%, DD-Galacturonic acid is 9.52%, apple pectin is 9.52%, bitter orange - *Citrus aurantium* is 4.76%, ginger - *Zingiber officinale* is 2.38%, *Radix Isatidis* is 2.38%, *Lindera aggregate* is 2.38%, woad - *Isatis indigotica* is 1.19%, *Torreya nucifera* is 1.19%, geranium extract - *Pelargonium* is 1.19%, Zinc is 1.19% and Sodium Selenite is 0.01%.

The advantage of the created food supplement is that it has anti-inflammatory, antimicrobial and immunomodulatory effect, acts against the penetration of external pathogens into the cell, maintains the functions of the immune system and increases the body's defences.

### Examples of embodiments of the utility model

The developed nutritional supplement to stimulate immunity, in a preferred embodiment, includes the following components in weight percent: liquorice *- Glycyrrhiza glabra* 11.91%, sweet wormwood - *Artemisia annua* 11.91%, quinine bark extract - *Cinchona* 11.91 %, hawthorn extract - *Crataegus* 9.52%, dandelion extract - *Taraxcum* 9.52%, vitamin C - Ascorbic acid 9.52%, DD-Galacturonic acid 9.52%, apple pectin 9.52%, bitter orange - *Citrus aurantium* 4.76%, ginger - *Zingiber officinale* 2.38%, *Radix Isatidis* 2.38%, *Lindera aggregate* 2.38%, woad - *Isatis indigotica* 1.19%, *Torreya nucifera* 1.19%, geranium extract - *Pelargonium* 1.19%, Zinc 1.19% and Sodium Selenite 0.01%.

Liquorice (*Glycyrrhiza glabra*) has a long history of use in the treatment of coughs and colds, as well as for the regulation of indigestion. Ammonium glycyrrhizinate, which is the active substance in liquorice, has an anti-inflammatory effect and is used to treat the damage caused by hepatitis B to the liver. In combination with vitamin C it also shows anti-viral action against various flu and other viruses.

Sweet wormwood (*Artemisia annua*) has anti-inflammatory and immunomodulatory effect and prevents the penetration of foreign pathogens into the cell.

The quinine contained in the quinine tree extract (*Cinchona*) alkalizes cell membranes, thus inhibiting viral replication.

Hawthorn extract (*Crataegus*) protects the cardiovascular system by taking care of the condition of the heart muscle, supports normal heart rhythm and has a beneficial effect on peripheral, coronary and cerebral blood vessels. Dandelion extract (*Taraxcum*) has a proven anti-viral effect by inhibiting the entry of viruses into host cells by inhibiting key viral enzymes.

Vitamin C (Ascorbic acid) is a vitamin with a strong antioxidant and immunostimulating effect.

D-D-Galacturonic acid helps lower serum cholesterol and triglycerides. In addition, it improves the digestive system, helps regulate body weight and blood sugar and strengthens the immune system.

Apple pectin helps lower serum cholesterol and triglycerides. Improves the digestive system, helps regulate body weight and blood sugar and strengthens the immune system.

Bitter orange (*Citrus aurantium*) has a proven antifungal effect, helps regulate body weight and thus strengthens the immune system.

Ginger (*Zingiber officinale*) is considered an effective remedy against nausea, diarrhea, colic and other stomach problems, as well as a painkiller for rheumatic diseases and menstrual pain. Strengthens the immune system.

*Radix isatidis* is an extract of the dried root of the plant *Isatis tinctorial L,* known for its immunostimulating, anti-infective and anti-inflammatory properties. The plant is widely used in traditional Chinese medicine to stimulate the body's immune system, fight bacterial and viral infections and accelerate recovery after illness.

*Lindera aggregate* has a proven anti-viral effect.

*Isatis indigotica* has a proven antibacterial, antiviral and anti-inflammatory effect.

*Torreya nucifera* has a proven antiviral effect.

Geranium extract (*Pelargonium*) has a proven antiviral effect by inhibiting the entry of viruses into host cells and also by inhibiting key viral enzymes.

Zinc increases resistance to infections and has antiviral activity.

Sodium Selenite is involved in the formation of selenoenzymes with antioxidant action. Reduces oxidative stress and increases immunity.

The created food supplement to stimulate immunity is produced as follows:
All components are in powder form. They are pre-tested for moisture content, which should be no more than 5-6%, and a microbial analysis is performed. The mixing is performed in reverse order of the percentage input, ie. it starts from the component with the lowest weight percentage to a higher one. In this case, sodium selenite is combined with zinc, then woad, *Thorium Nucifera* and geranium extract are added. Mixing is carried out in a turbulent mixer at 18 °C and humidity of 28-31%. The next group of low-percentage ingredients is added sequentially under the same conditions - bitter orange, ginger, *Radix Isatidis* and *Linder Aggregate,* and so on until the end, when higher percentage ingredients are added to the mix.

Binders such as microcrystalline cellulose, stearate, silica, etc. are added to the finished mixture. The final mixture is mixed under the same conditions for about 30-40 minutes, then sealed in dry containers under pressure of dry nitrogen and provided in portions for dosing to capsule dispensers. The ready dosed capsules are stored at a temperature of about 18-20 °C until packed in blisters. The whole process is carried out in a microbially clean environment, according to GMP practice. The finished blister product is submitted for packaging, marking and shipping.

## Claims

1. **Anti-viral, anti-microbial and stimulating immunity food supplement, characterized in that** it includes the following components in weight percentages:
- liquorice - *Glycyrrhiza glabra* from 1,19% to 47,61%,
- sweet *wormwood - Artemisia annua* from 1,19% to 47,61%,
- quinine bark extract - *Cinchona* from 1,19% to 47,61%,
- hawthorn extract - *Crataegus* from 1,19% to 47,61%,
- dandelion extract - *Taraxcum* from 1,19% to 47,61%,
- vitamin C - Ascorbic acid from 1,19% to 47,61%,
- D-D- Galacturonic acid from 1,19% to 47,61%,
- apple pectin from 1,19% to 47,61%,
- bitter orange - *Citrus aurantium* from 1,19% to 47,61%,
- ginger - *Zingiber officinale* from 0,23% to 11,9%,
- *Radix isatidis* from 0,23% to 11,9%,
- *Lindera aggregate* from 0,23% to 11,9%,
- woad - *Isatis indigotica* from 0,23% to 9,52%,
- *Torreya nucifera* from 0,23% to 9,52%,
- geranium extract - *Pelargonium* from 0,23% to 9,52%,
- Zinc from 0,23% to 2,38% and
- Sodium Selenite from 0,00119% to 0,04762%.

2. **Anti-viral, anti-microbial and stimulating immunity food supplement,** according to claim 1, **characterized in that**
- liquorice - *Glycyrrhiza glabra* is 11,91%,
- sweet wormwood *- Artemisia annua* is 11,91%,
- quinine bark extract - *Cinchona* is 11,91%,
- hawthorn extract - *Crataegus* is 9,52%,
- dandelion extract - *Taraxcum* is 9,52%,
- vitamin C - Ascorbic acid is 9,52%,
- D-D- Galacturonic acid is 9,52%,
- apple pectin is 9,52%,
- bitter orange - *Citrus aurantium* is 4,76%,
- ginger - *Zingiber officinale* is 2,38%,
- *Radix isatidis* is 2,38%,
- *Lindera aggregate* is 2,38%,
- woad - *Isatis indigotica* is 1,19%,
- *Torreya nucifera* is 1,19%,
- geranium extract - *Pelargonium* is 1,19%,
- Zinc is 1,19% and
- Sodium Selenite is 0,01%.

## Patentansprüche

1. **Nahrungsergänzungsmittel mit antiviraler, antimikrobieller Wirkung und zur Stimulierung der Immunität, dadurch gekennzeichnet, dass es folgende Bestandteile in Gewichtsprozenten enthält:**
- Lakritze - *Glycyrrhiza glabra* von 1,19% bis zum 47,61%,
- süßer Wermut - *Artemisia annua* von 1,19% bis zum 47,61%,
- Chininrindenextrakt - *Cinchona* von 1,19% bis zum 47,61%,
- Weißdorn-Extrakt - *Crataegus* von 1,19% bis zum 47,61%,
- Löwenzahnextrakt - *Taraxcum* von 1,19% bis zum 47,61%,
- Vitamin C - *Ascorbic acid* von 1,19% bis zum 47,61%,
- D-D- Galakturonsäure von 1,19% bis zum 47,61%,
- Apfelpektin von 1,19% bis zum 47,61%,
- bittere Orange - *Citrus aurantium* von 1,19% bis zum 47,61%,
- Ingwer - *Zingiber officinale* von 0,23% bis zum 11,9%,
- *Radix isatidis* von 0,23% bis zum 11,9%,
- *Lindera aggregate* von 0,23% bis zum 11,9%,
- Sichel färben - *Isatis indigotica* von 0,23% bis zum 9,52%,
- *Torreya nucifera* von 0,23% bis zum 9,52%,
- Geranieextrakt - *Pelargonium* von 0,23% bis zum 9,52%,
- Zink - *Zinc* von 0,23% bis zum 2,38% und
- Natriumselenit - *Sodium Selenite* von 0,00119% bis zum 0,04762%.

2. **Nahrungsergänzungsmittel mit antiviraler, antimikrobieller und immunstimulierender Wirkung, nach Anspruch 1, dadurch gekennzeichnet, dass**
- Lakritze - Glycyrrhiza glabra 11,91%,
- süßer Wermut - Artemisia annua 11,91%,
- Chininrindenextrakt - Cinchona 11,91%,
- Weißdorn-Extrakt - Crataegus 9,52%,
- Löwenzahnextrakt - Taraxcum 9,52%,
- Vitamin C - Ascorbic acid 9,52%,
- D-D- Galakturonsäure 9,52%,
- Apfelpektin 9,52%,
- bittere Orange - Citrus aurantium 4,76%,
- Ingwer - Zingiber officinale 2,38%,
- Radix isatidis 2,38%,
- Lindera aggregate 2,38%,
- Sichel färben - Isatis indigotica 1,19%,
- Torreya nucifera 1,19%,
- Geranieextrakt - Pelargonium 1,19%,
- Zink - Zinc 1,19% und
- Natriumselenit - Sodium Selenite 0,01% beträgt.

## Revendications

1. **Complément alimentaire à action antivirale, antimicrobienne et stimulant l'immunité, caractérisé par le fait qu'**il comprend les composants suivants en pourcentage de poids :
- réglisse - *Glycyrrhiza glabra* de 1,19% à 47,61%,
- absinthe douce - *Artemisia annua* de 1,19% à 47,61%,
- extrait d'écorce de quinine - *Cinchona* de 1,19% à 47,61%,
- extrait d'aubépine - *Crataegus* de 1,19% à 47,61%,
- extrait de pissenlit - *Taraxcum* de 1,19% à 47,61%,
- Vitamine C - Ascorbic acid de 1,19% à 47,61%,
- Acide D-D-Galacturonique de 1,19% à 47,61%,
- pectine de pomme de 1,19% à 47,61%,
- orange amère - *Citrus aurantium* de 1,19% à 47,61%,
- gingembre - *Zingiber officinale* de 0,23% à 11,9%,
- *Radix isatidis* de 0,23% à 11,9%,
- *Lindera aggregata* de 0,23% à 11,9%,
- guède - *Isatis indigotica* de 0,23% à 9,52%,
- *Torreya nucifera* de 0,23% à 9,52%,
- extrait de géranium - *Pelargonium* de 0,23% à 9,52%,
- Zinc de 0,23% à 2,38% et
- Sélénite de sodium de 0,00119% à 0,04762%.

2. **Complément alimentaire à action antivirale, antimicrobienne et stimulant l'immunité** selon la revendication 1**, caractérisé par le fait que**
- la réglisse - *Glycyrrhiza glabra* est 11,91%,
- l'absinthe douce - *Artemisia annua* est 11,91%,
- l'extrait d'écorce de quinine - *Cinchona* est 11,91%,
- l'extrait d'aubépine - *Crataegus* est 9,52%,
- l'extrait de pissenlit - *Taraxcum* est 9,52%,
- le Vitamine C - Ascorbic acid est 9,52%,
- l'Acide D-D-Galacturonique est 9,52%,
- la pectine de pomme est 9,52%,
- l'orange amère - *Citrus aurantium* est 4,76%,
- le gingembre - *Zingiber officinale* est 2,38%,
- le *Radix isatidis* est 2,38%,
- la *Lindera aggregata* est 2,38%,
- le guède - *Isatis indigotica* est 1,19%,
- la *Torreya nucifera* est 1,19%,
- l'extrait de géranium - *Pelargonium* est 1,19%,
- le Zinc est 1,19% et
- le Sélénite de sodium est 0,01%.
